# EUROPEAN PATENT APPLICATION

(11) **EP 4 454 608 A1**
(43) Date of publication of application: **30.10.2024**
(21) Application number: 22909611.0
(22) Date of filing: 18.11.2022
(51) Int. Cl.: A61F 2/24

(54) **VALVE CLAMPING APPARATUS**

(30) Priority: 23.12.2021 CN 202111592201
(71) Applicant: Hangzhou Valgen Medtech Co., Ltd., Hangzhou, Zhejiang 310051 (CN)
(72) Inventor: ZHANG, Tingchao, Hangzhou, Zhejiang 310051 (CN); ZHENG, Xianzhang, Hangzhou, Zhejiang 310051 (CN); LIANG, Huaguang, Hangzhou, Zhejiang 310051 (CN); JIANG, Wei, Hangzhou, Zhejiang 310051 (CN)
(74) Representative: Inchingalo, Simona
(86) International application number: PCT/CN2022/132838
(87) International publication number: WO 2023/116295

(57) **Abstract**

A valve clamping apparatus (1) includes a supporting portion (10) having a certain length, a clamping portion (20) including a pair of clamping arms (21) that can be relatively opened and closed, and an adjusting portion (30). The adjusting portion (30) is arranged outside the supporting portion (10), the adjusting portion (30) is deformable, and the adjusting portion (30) is a three-dimensional structure having a certain length, width, and thickness. When the pair of clamping arms (21) is closed relative to the adjusting portion (30), the adjusting portion (30) deforms. A deformation degree of the adjusting portion (30) in a thicknesswise direction is greater than a deformation degree of the adjusting portion (30) in a lengthwise direction, which is greater than a deformation degree of the adjusting portion (30) in a widthwise direction.

## Description

### TECHNICAL FIELD

The present application relates to the field of medical devices, and in particular, to a valve clamping device.

### BACKGROUND

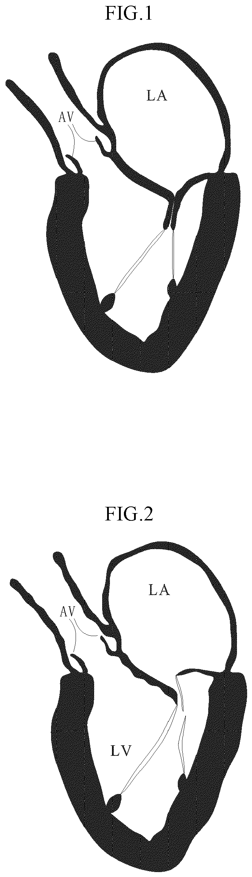

Mitral valve is a one-way valve located between the left atrium and the left ventricle of the heart. A normal and healthy mitral valve can control the blood flow from the left atrium to the left ventricle, while preventing blood from flowing from the left ventricle to the left atrium. The mitral valve includes a pair of leaflets, called the anterior leaflet and the posterior leaflet, as shown in FIG. 1, wherein AV is the abbreviation of aortic valve, LA is the abbreviation of left atrium, and LV is the abbreviation of left ventricular. During the cardiac systole, when the edges of the anterior leaflet and the posterior leaflet of the mitral valve are aligned, the mitral valve can be completely closed to prevent blood from flowing from the left ventricle to the left atrium. As shown in FIG. 2 , when the leaflets of the mitral valve or their related structures undergo organic or functional changes, the anterior and posterior leaflets of the mitral valve are not properly aligned, as a result, when the heart contracts, the mitral valve cannot be completely closed, causing blood to flow back from the left ventricle to the left atrium, thereby causing a series of pathological and physiological changes, known as "mitral regurgitation".

Surgical procedures such as edge-to-edge suturing are usually used to treat mitral regurgitation. However, this type of surgery has disadvantages such as complex procedure, high cost, severe trauma, high risk of complications, long hospital stay, and painful recovery process for patients.

Currently, there is a valve clamping device, which clamps the anterior and posterior leaflets of the mitral valve respectively through a pair of openable and closable clips, and then brings the two clips closer together, thereby pulling the anterior and posterior leaflets toward each other to form an "8"-shaped structure, so that the original large leaflet gap becomes two smaller gaps, thereby eliminating or reducing mitral regurgitation. However, due to the gap at the connection between the two clips, blood can still flow back to the left atrium through the gap, thereby affecting the treatment.

### SUMMARY

The present application provides a valve clamping device includes a supporting portion having a certain length; a clamping portion comprising a pair of clamping arms that can be opened and closed relative to each other; and an adjusting portion. Wherein the adjusting portion is arranged outside the supporting portion, the adjusting portion is deformable, the adjusting portion is a three-dimensional structure with a certain length, width and thickness, and the pair of the clamping arms are closed relative to the adjusting portion so that the adjusting portion is deformed, and the deformation degree of the adjusting portion in the thicknesswise direction>the deformation degree of the adjusting portion in the lengthwise direction>the deformation degree of the adjusting portion in the widthwise direction.

In some embodiments, a deformation rate of the adjusting portion in the thicknesswise direction ranges from 25% to 65%.

In some embodiments, a deformation rate of the adjusting portion in the lengthwise direction ranges from 10% to 20%.

In some embodiments, a deformation rate of the adjusting portion in the widthwise direction ranges from 3% to 12%.

In some embodiments, the adjusting portion is made of shape memory material and has been heat-set.

In some embodiments, the adjusting portion includes a mesh structure, and a porosity of the mesh structure ranges from 30% to 80%.

In some embodiments, the adjusting portion is made of shape memory material and has been heat-set.

In some embodiments, the mesh structure is woven by a plurality of filaments, and a diameter of the filaments ranges from 0.06 mm to 0.20 mm.

In some embodiments, the weaving manner of the filament is selected from at least one of a single-strand weaving manner, a double-strand weaving manner or a mixed weaving manner of single and double strands.

In some embodiments, one end of the adjusting portion is fixedly connected with the supporting portion.

In some embodiments, the valve clamping device further includes a driving part configured to drive the relative opening and closing of the pair of clamping arms.

In some embodiments, the driving part includes a pair of connecting rods and an elastic closing unit, one end of each of the connecting rods is rotatably connected to one of the clamping arms, and the other end of the each connecting rods is connected to the supporting portion, one end of the elastic closing unit is connected to the connecting rod, and the other end of the elastic closing unit is connected to the clamping arm.

In some embodiments, the driving part includes a pair of connecting rods, a driving shaft and a connecting seat, one end of each of the connecting rods is rotatably connected to one of the clamping arms, and the other end of each of the connecting rods is rotatably connected to the connecting seat, one end of the drive shaft is connected to the connecting seat, and the other end of the drive shaft is movably fitted in the supporting portion.

In some embodiments, a surface of the clamping arm facing the adjusting portion is provided with a plurality of protrusions, and when the clamping arm is closed relative to the adjusting portion, the protrusions abut against the adjusting portion.

In some embodiments, the adjusting portion has at least one set of first barbs provided on an area thereof opposite to the clamping arm, and the length of the first barbs ranges from 0.3mm to 3mm.

In some embodiments, the first barb is inclined in a direction toward a distal end of the supporting portion.

In some embodiments, the included angle between the first barb and the tangent at the connection with the outer surface of the adjusting portion is less than 90°.

In some embodiments, along the direction of getting away from the distal end of the supporting portion, the length of the first barb gradually increases.

In some embodiments, the valve clamping device further includes a gripper, the gripper includes a pair of elastic arms, each of the elastic arms is located between one of the clamping arms and the adjusting portion, and the elastic arm and the clamping arm cooperate to clamp a leaflet.

In some embodiments, when both the clamping arms and the gripper are in the unfolded state, the angle between the two clamping arms is smaller than the angle between the two elastic arms.

In some embodiments, the elastic arm is provided with at least one set of second barbs on the surface facing the clamping arm.

In certain embodiments, the valve clamping device of the present disclosure sets the size relationship of the deformation degree of the adjusting portion in the widthwise direction, lengthwise direction and thicknesswise direction, so that the adjusting portion can provide appropriate radial support force for the clamping arm, avoid the valve clamping device from falling off, prevent the leaflets from being over-pulled, prevent the leaflets from being subjected to stress concentration and causing leaflet injuries, improve the fatigue resistance of the clamping device, and effectively block the central regurgitation of the clamping gap.

Other aspects and advantages of the present disclosure will be partially given in the following description, and partially will become obvious from the following description, or will be understood through the practice of the present disclosure.

### BRIEF DESCRIPTION OF THE ACCOMPANYING DRAWINGS

The above and/or additional aspects and advantages of the present disclosure will become obvious and easy to understand from the description of the embodiments in conjunction with the following drawings, in which:
FIG.1 is a schematic view of the mitral valve in a normal state.
FIG.2 is a schematic view of a structure in which a heart disease occurs and the mitral valve cannot close.
FIG.3 is a schematic view of a valve clamping device clamping a mitral valve according to an embodiment of the present application.
FIG.4 is a front view of a valve clamping device in an unfolded state according to some embodiments of the present disclosure.
FIG.5 is a schematic view of an increase in the porosity of a mesh structure of an adjusting portion of the valve clamping device relative to that shown in FIG. 4.
FIG.6 is a schematic view of a decrease in the porosity of a mesh structure of an adjusting portion of the valve clamping device relative to that shown in FIG. 4.
FIG.7 is a schematic diagram of an increase in the diameter of the filament of a mesh structure of an adjusting portion of the valve clamping device relative to that shown in Fig. 4.
FIG.8 is schematic view of a mesh structure of an adjusting portion of a valve clamping device according to an embodiment of the present disclosure.
FIG.9 is a three-dimensional view of a valve clamping device including a supporting portion, a clamping portion and a gripper in an unfolded state according to some embodiments of the present disclosure.
FIG. 10 is a three-dimensional view of a valve clamping device including a supporting portion, a clamping portion and a gripper in a closed state according to other embodiments of the present disclosure.
FIG. 11 a front view of the valve clamping device in an unfolded state according to other embodiments of the present disclosure.
FIG. 12 is a schematic diagram of a single-strand weaving manner of a mesh structure of an adjusting portion of a valve clamping device according to some embodiments of the present disclosure.
FIG. 13 is a schematic diagram of a single-strand and double-strand weaving manner of a mesh structure of an adjusting portion of a valve clamping device according to some embodiments of the present disclosure.
FIG. 14 is a schematic diagram of a double-strand weaving manner of a mesh structure of an adjusting portion of a valve clamping device according to some embodiments of the present disclosure.
FIG. 15 is a front view of a valve clamping device including a supporting portion, a clamping portion and a gripper in an unfolded state according to other embodiments of the present disclosure.
FIG. 16 is a front view of the valve clamping device of the embodiment shown in FIG. 15.

Figure markings: valve clamping device 1; supporting portion 10; clamping portion 20; clamping arm 21; protrusion 212; adjusting portion 30; first barb 31; mesh structure 32; filament 320; driving part 40; connecting rod 41; elastic closing unit 42; driving shaft 52; connecting seat 53; gripper 60; elastic arm 602; second barb 61.

### DETAILED DESCRIPTION OF ILLUSTRATED EMBODIMENTS

The following describes in detail the embodiments of the present disclosure, examples of which are shown in the accompanying drawings, wherein the same or similar reference numerals throughout represent the same or similar elements or elements having the same or similar functions. The embodiments described below with reference to the accompanying drawings are exemplary and are only used to explain the present disclosure, and should not be construed as limiting the present disclosure.

It should be noted that in the field of interventional medical devices, the proximal end refers to the end closer to the operator, and the distal end refers to the end farther from the operator.

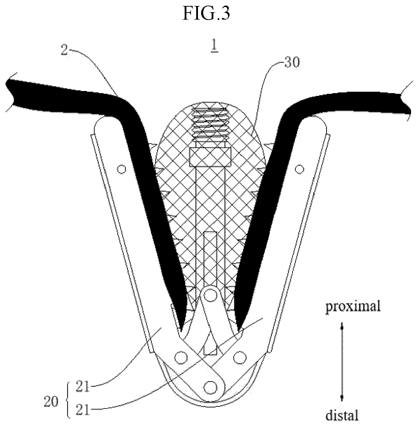

As shown in FIGs. 1 to 4, the valve clamping device 1 according to the embodiment of the present disclosure includes a supporting portion 10, a clamping portion 20 and an adjusting portion 30.

In detail, the supporting portion 10 has a certain length; the clamping portion 20 includes a pair of clamping arms 21 that can be relatively opened and closed; the adjusting portion 30 is sleeved outside the supporting portion 10, the adjusting portion 30 is deformable, and the adjusting portion 30 is a three-dimensional structure with a certain length, width and thickness. The pair of clamping arms 21 are closed relative to the adjusting portion 30 to deform the adjusting portion 30, and the deformation degree of the adjusting portion 30 in the thicknesswise direction > the deformation degree of the adjusting portion 30 in the lengthwise direction > the deformation degree of the adjusting portion 30 in the widthwise direction.

The proximal end of the supporting portion 10 is configured to be detachably connected to the delivery system, so that the valve clamping device 1 can be delivered to the patient's body through a catheter or through the heart apex, and can be implanted for a long time after being released from the delivery system. The distal end of the supporting portion 10 is configured to connect the clamping portion 20, and can serve as a fulcrum for the two clamping arms 21 to open and close around the supporting portion 10 and the adjusting portion 30. The supporting portion 10 can be a circular tube, a square tube, an oblate tube or a combination of multiple tubes with two end faces axially connected. The present embodiment adopts a circular tube. It should be noted that the supporting portion 10 has a certain length, which means that the supporting portion 10 has a certain length in the axial direction, the supporting portion 10adjusting portion can be sleeved on the supporting portion 10, and the supporting portion 10 can also be assembled and connected with other structures to meet the "certain length".

One end of the clamping portion 20 is rotatably connected to one end of the supporting portion 10, so that the clamping portion 20 can be opened or closed around the supporting portion 10 and the adjusting portion 30. In some embodiments, the clamping portion 20 includes a pair of clamping arms 21 that can be relatively opened and closed, and one end of each clamping arm 21 is rotatably connected to one end of the supporting portion 10, so that the clamping arm 21 can be relatively opened and closed and close to or away from the supporting portion 10. The adjusting portion 30 is sleeved outside the supporting portion 10, so when the clamping arm 21 is relatively opened and closed, it can be close to or away from the adjusting portion 30, thereby fixing the leaflets between the clamping arm 21 and the adjusting portion 30. Since the adjusting portion 30 is deformable, when the clamping arm 21 is closed, the adjusting portion 30 is compressed to deform, and at this time, the adjusting portion 30 provides a reaction force (also called a support force) for the clamping arm 21, which serves as the clamping force of the valve clamping device 1 to clamp the valve leaflets. Since the adjusting portion 30 is a three-dimensional structure with a certain length, width and thickness, when it is compressed by the clamping arm 21, it deforms to different degrees in different directions, thereby affecting the support force that the adjusting portion 30 can provide to the clamping arm 21, and further affecting the clamping force and fatigue resistance of the valve clamping device 1.

At least one end of the adjusting portion 30 is fixedly connected to the supporting portion 10. In one embodiment, the distal end of the adjusting portion 30 is fixed, and the proximal end is not fixed as a free end, so that when the adjusting portion 30 is radially compressed by the clamping arm 21, the proximal end of the adjusting portion 30 can move axially relative to the supporting portion 10, so that in the process of the clamping arm 21 closing around the adjusting portion 30 to clamp the leaflets, the adjusting portion 30 shrinks in thickness, elongates in length, and increases in width. It can be understood that in other embodiments, both ends of the adjusting portion 30 are fixed on the supporting portion 10, and when the adjusting portion 30 is compressed by the clamping arm 21, although the axial movement of the fixed end of the adjusting portion 30 is limited, the main body of the adjusting portion 30 will still shrink in the radial direction due to being compressed by the clamping portion 20, and the part of the main body of the adjusting portion 30 close to the fixed end will extend upward (towards the end of the clamping arm 21) or downward (towards the root of the clamping arm 21), and exceed the fixed end, presenting an inverted cone, at this time, the adjusting portion 30 still has the phenomenon of reduced thickness, elongated length, and increased width. In the present disclosure, the methods of fixing the adjusting portion 30 to the supporting portion 10 include but are not limited to welding, bonding, threaded connection, snap-fit connection, etc.

As shown in FIG. 1 , in the present disclosure, the direction in which a pair of clamping arms 21 can be relatively opened and closed is defined as the thicknesswise direction of the adjusting portion 30, the direction perpendicular to the plane of the moving trajectory of the clamping arms 21 is defined as the widthwise direction of the adjusting portion 30, and the direction in the same direction as the axial length of the supporting portion 10 is defined as the lengthwise direction of the adjusting portion 30. The two clamping arms 21 are closed relative to the adjusting portion 30, so that the adjusting portion 30 is compressed and deformed, and the adjusting portion 30 changes from the original more three-dimensional sphere to a flatter ellipsoid. At this time, the thickness of the adjusting portion 30 is reduced, and the length and width are increased.

Wherein, the original length of the adjusting portion 30 is L1, the length after deformation is L2, and the deformation rate ΔL in the lengthwise direction is defined as: ΔL=(L2-L1)/L1. The original width of the adjusting portion 30 is W1, the width after deformation is W2, and the deformation rate ΔW in the widthwise direction is defined as: ΔW=(W2-W1)/W1. The original thickness of the adjusting portion 30 is T1, the thickness after deformation is T2, and the deformation rate ΔT in the thicknesswise direction is defined as: ΔT=(T1-T2)/T1. In the present disclosure, the deformation rate of the adjusting portion 30 is defined as: ΔS=ΔL*ΔW*ΔT.

When the valve clamping device 1 is adapted to clamp the leaflets of the mitral valve, when the two clamping arms 21 are closed relative to the adjusting portion 30, the anterior leaflet of the mitral valve is located between one clamping arm 21 and one side of the adjusting portion 30 in the thicknesswise direction, and the posterior leaflet of the mitral valve is located between the other clamping arm 21 and the other side of the adjusting portion 30 in the thicknesswise direction. The anterior leaflet and the posterior leaflet are tightly pulled between the adjusting portion 30 and the clamping arm 21 through the deformation of the adjusting portion 30, thereby reducing or eliminating the leaflet gap and reducing mitral regurgitation. It can be seen that the deformation degree of the adjusting portion 30 in different directions directly affects the clamping performance of the clamping arm 21 on the leaflet and the degree of pulling the leaflet. In the present disclosure, since the deformation degree of the adjusting portion 30 in the thicknesswise direction is greater than the deformation degree of the adjusting portion 30 in the lengthwise direction and the deformation degree of the adjusting portion 30 in the widthwise direction, the deformed adjusting portion 30 can provide appropriate radial support force for the clamping arm 21, prevent the leaflet from being excessively pulled, prevent the leaflet from being subjected to stress concentration and causing leaflet injuries, and avoid affecting the long-term fatigue performance of the clamping arm 21. In addition, since the adjusting portion 30 is filled between the clamping arms 21, it can also reduce the residual backflow in the gap between the two clamping arms 21. At the same time, it can also deform in real time with the pulsation of the leaflet to adapt to and adjust the leaflet gap.

According to a valve clamping device 1 of an embodiment of the present disclosure, the adjusting portion 30 is made of a shape memory material and is subjected to a heat-set treatment, so that the adjusting portion 30 can have a specific shape, and when subjected to an external force, such as when compressed by the clamping arm 21, it can be deformed and tend to restore its original shape, thereby providing support for the clamping arm 21. The material of the supporting portion 10 and the clamping arm 21 can be a biocompatible metal material such as stainless steel, cobalt-chromium alloy and nickel-titanium alloy, and in some embodiments, stainless steel, but the present disclosure is not limited thereto.

The deformation rate of the adjusting portion 30 in the thicknesswise direction ranges from 25% to 65%. Since the leaflets are clamped between the two sides of the clamping arm 21 and the adjusting portion 30 in the thicknesswise direction after the clamping arm 21 is closed, the deformation degree of the adjusting portion 30 in the thicknesswise direction directly affects the support force provided to the clamping arm 21 and the leaflets, and is directly related to the clamping force of the valve clamping device 1. If the deformation rate is too small, the support force is insufficient, resulting in insufficient clamping force of the valve clamping device 1, and it is easy to fall off the leaflets; if the deformation rate is too large, the leaflets may be over-pulled and damaged, or the leaflets may be perforated due to stress concentration. Therefore, in this embodiment, the deformation rate of the adjusting portion 30 in the thicknesswise direction ranges from 25% to 65%, which can provide appropriate support and clamping force, and avoid leaflet injuries caused by excessive pulling of the leaflets.

In one embodiment of the present disclosure, the deformation rate of the adjusting portion 30 in the lengthwise direction ranges from 10% to 20%. In some embodiments, since only one end of the adjusting portion 30 is fixed on the supporting portion 10, after the clamping arm 21 is closed, the free end of the adjusting portion 30 that is not fixed will produce axial movement relative to the supporting portion 10, causing the adjusting portion 30 to deform in the lengthwise direction. If the degree of deformation is too small, it means that the radial support force provided by the adjusting portion 30 is small and cannot continuously clamp the leaflet; if the degree of deformation is too large, it means that the free end of the adjusting portion 30 extends beyond the end of the supporting portion 10 or the clamping arm 21, and the exposed free end may cause leaflet wear or blood accumulation to produce thrombus. In other embodiments, both ends of the adjusting portion 30 are fixed on the supporting portion 10, after the clamping arm 21 is closed, the main body of the adjusting portion 30 produces axial movement and extends beyond the fixed end, presenting an inverted cone, at this time, the adjusting portion 30 also undergoes deformation in the lengthwise direction, and the degree of deformation will also affect the support force of the adjusting portion 30 and whether it is exposed.

In one embodiment of the present disclosure, the deformation rate of the adjusting portion 30 in the widthwise direction ranges from 3% to 12%. When the clamping arm 21 is closed, the adjusting portion 30 is deformed under pressure, increases in the widthwise direction and protrudes to the outside of the clamping arm 21. Therefore, the degree of deformation of the adjusting portion 30 in the widthwise direction will affect the overall outer diameter of the valve clamping device 1 after closing. Referring to the gap between the inner diameter of the tube body of the existing delivery system and the outer diameter of the implant, in order to facilitate loading in the catheter of the delivery system, the deformation of the adjusting portion 30 in the widthwise direction should not be too large. In this embodiment, the deformation rate of the adjusting portion 30 in the widthwise direction ranges from 3% to 12%.

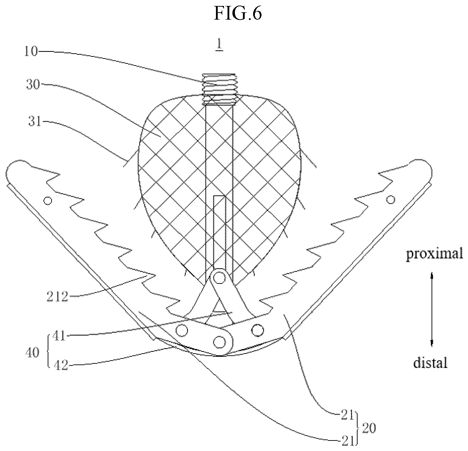

As shown in FIGs. 1 to 6, in one embodiment of the present disclosure, the adjusting portion 30 includes a mesh structure 32, and the porosity of the mesh structure 32 ranges from 30% to 80%. The appropriate porosity can not only enable the adjusting portion 30 to effectively fill the gap between the clamping arms 21 of the valve clamping device 1 (i.e., the gap between the leaflets each time the leaflets are closed after being clamped), but also affect to a certain extent the degree of deformation of the adjusting portion 30 after being compressed and the support force it provides for the clamping arms 21. It should be noted that the "porosity" in the present disclosure refers to the percentage of the mesh area of the mesh structure 32 to the entire surface area of the adjusting portion 30. As shown in FIG.s 4, 5 and 6, the mesh structures 32 with different porosities are represented respectively. The ability of the mesh structure 32 to block the gap between the leaflets is related to its porosity. The setting of the porosity needs to satisfy the requirement of both allowing blood flow to pass and blocking thrombus. Specifically, if the porosity is too large, the blood permeability is too large, and the backflow of the clamped leaflet gap cannot be effectively blocked; if the porosity is too small, it is easy to cause thrombus accumulation inside the valve clamping device 1. In addition, the porosity will also affect the deformation rate of the mesh structure 32. A larger porosity means that the mesh area of the adjusting portion 30 is larger, the deformation rate when subjected to pressure is too large, and the radial support force is too large; while a smaller porosity means that the mesh area of the mesh structure 32 is smaller, the deformation rate when subjected to pressure is too small, and the radial support force provided is insufficient. Therefore, a suitable porosity can enable the adjusting portion 30 to provide a suitable radial support force for the clamping arm 21. Among them, "radial support force" refers to the tension provided to the outside by the adjusting portion 30 when it is compressed on the radial plane, which affects the clamping force and fatigue resistance of the valve clamping device 1 on the leaflets: if the radial support force is too small, it cannot provide sufficient clamping force, the valve clamping device 1 cannot effectively clamp the leaflets for a long time, the leaflets are easy to slip off, and the fatigue resistance of the valve clamping device 1 is not met; if the radial support force is too large, although it can provide sufficient clamping force, the clamping arm 21 is subjected to a large reverse force for a long time, and is easy to deform or even break, which also affects the fatigue resistance of the valve clamping device 1. Therefore, it is necessary to set a suitable porosity so that the adjusting portion 30 can effectively fill the gap between the clamping arms 21 of the valve clamping device 1 (i.e., the gap between the leaflets each time the leaflets are clamped and closed), and the adjusting portion 30 is deformed to a certain extent after being compressed to provide a suitable radial support force for the clamping arms 21, and the anti-fatigue performance of the valve clamping device 1 is not affected. In the present disclosure, the porosity of the mesh structure 32 of the adjusting portion 30 ranges from 30% to 80%.

The mesh structure 32 can be woven from filaments 320, or cut from a tube. Of course, there are other implementation methods, which are not limited by the present disclosure.

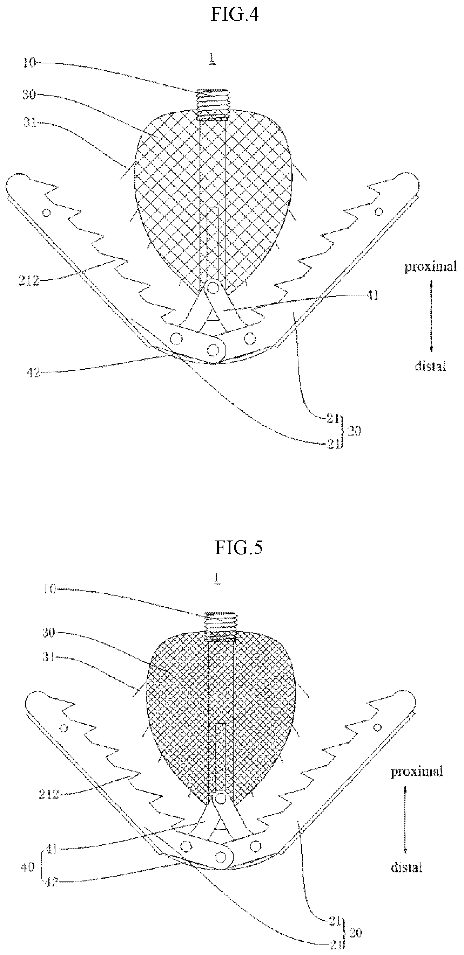

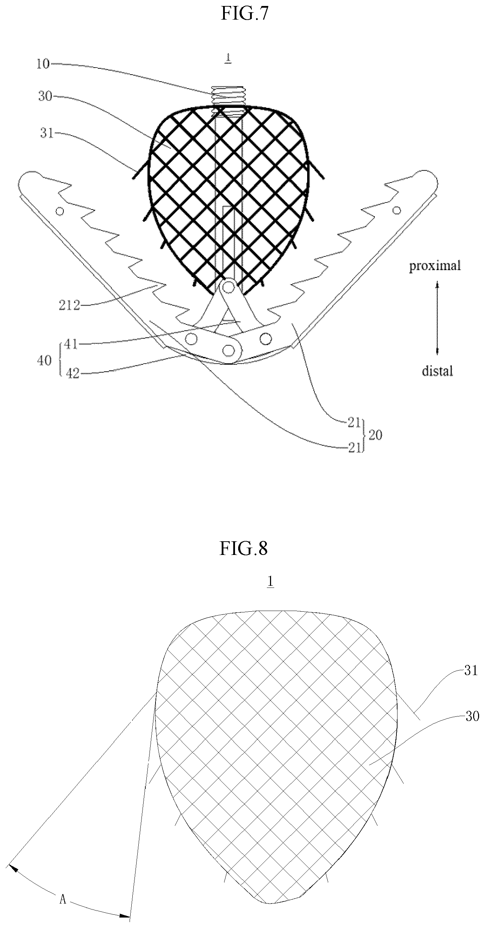

In one embodiment of the present disclosure, the mesh structure 32 is woven from a plurality of filaments 320, and the diameter of the filaments 320 ranges from 0.06 mm to 0.20 mm, as shown in FIGs. 4 and 8, which respectively show mesh structures 32 with different diameters. Under the condition of the same porosity, if the diameter is too small, the braided filament is easy to break, affecting the safety of the valve clamping device 1; if the diameter is too large, it will not only increase the difficulty of braiding, but also cause excessive radial support force, although it can provide sufficient clamping force, the clamping arm 21 is subjected to a large reverse force for a long time, affecting the anti-fatigue performance. In the present disclosure, the diameter range of the filament 320 is 0.06mm to 0.20mm, so that the filament 320 is easy to braid, has sufficient strength to prevent breakage, and can provide appropriate radial support force. Among them, the material of the filament 320 is selected from biocompatible metal materials such as nickel-titanium alloy, stainless steel, cobalt-chromium alloy, etc., and in some embodiments, it is nickel-titanium alloy.

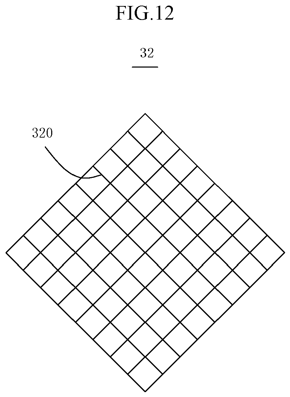

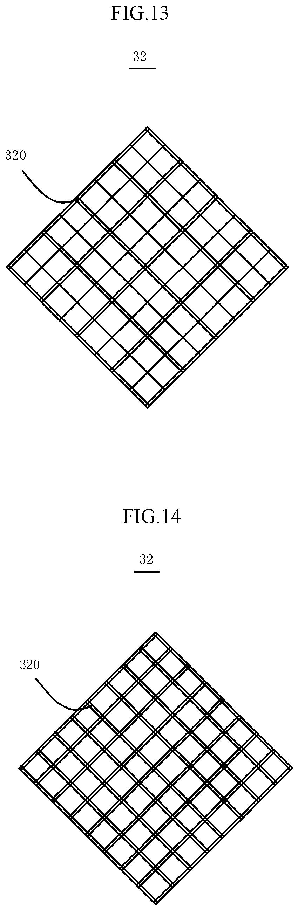

As shown in FIGs. 12-14, in one embodiment of the present disclosure, the weaving manner of the filaments 320 is selected from at least one of a single-strand weaving manner, a double-strand weaving manner, or a single-strand mixed weaving manner. Among them, when single-strand weaving is used, the mesh obtained by weaving is smaller and denser, and a woven mesh with a smaller porosity can be obtained, that is, the porosity is relatively small, but the radial support force is also relatively small; single-strand mixed weaving can combine woven filaments with different diameters for weaving, and a woven mesh with different porosities but with opposite radial support forces can be produced; when double-filament weaving is used, the mesh obtained by weaving will be relatively larger, that is, the porosity is relatively large, but the radial support force is also correspondingly large. In this way, the diversity of the structural schemes of the mesh structure 32 can be increased, and a suitable weaving manner can be selected according to the target parameters to meet the model specification requirements of the valve clamping device 1 suitable for mitral valves with different anatomical structures.

As shown in FIGs. 1-6 and FIGs. 8-10, according to the valve clamping device 1 of the embodiment of the present disclosure, the valve clamping device 1 also includes a driving unit 40, and the driving unit 40 is configured to drive the relative opening and closing of a pair of clamping arms 21. When the clamping arms 21 of the valve clamping device 1 are in the collapse state and delivered, the driving unit 40 drives the pair of clamping arms 21 to open and capture the leaflets, and then drives the pair of clamping arms 21 to close by the driving unit 40, so as to clamp the leaflets between the clamping arms 21 and the adjusting portion 30.

As shown in FIGs. 1-6 and 8, in one embodiment of the present disclosure, the driving unit 40 includes a pair of connecting rods 41 and an elastic closing unit 42, one end of each connecting rod 41 is rotatably connected to a clamping arm 21, the other end of each connecting rod 41 is connected to the supporting part 10, one end of the elastic closing unit 42 is connected to the connecting rod 41, and the other end of the elastic closing unit 42 is connected to the clamping arm 21. The elastic closing unit 42 is configured to keep the clamping arm 21 closed relative to the adjusting portion 30 in a natural state, that is, to provide a closing force of the valve clamping device 1, so that the clamping arm 21 is kept in a normally closed state after the clamping arm 21 captures the leaflets, and the clamping arm 21 is prevented from accidentally opening.

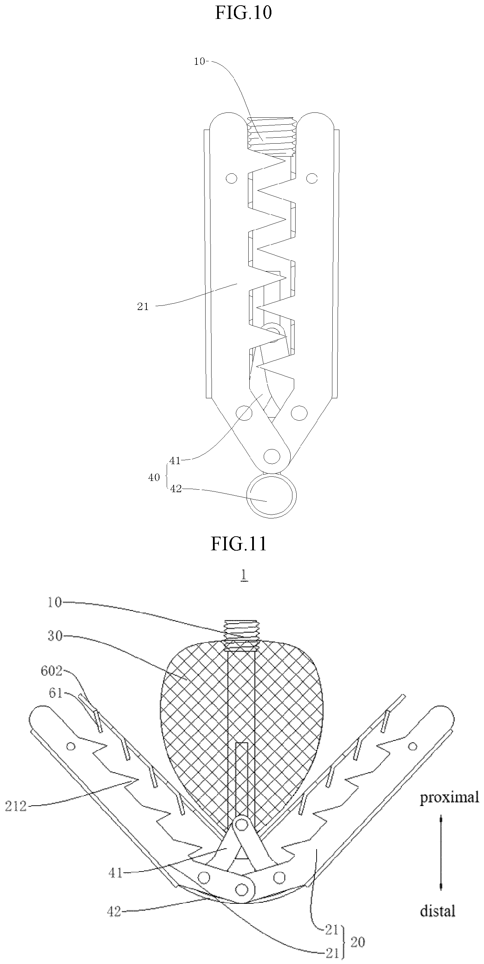

In some embodiments, the elastic closing force of the elastic closing unit 42 ranges from 3 to 12 N. If the closing force is too small, the leaflets cannot be effectively clamped and are easy to slip, while if the closing force is too large, the leaflets may be injuried. Please refer to FIGs. 1 to 6, the elastic closing unit 42 is a spring, and the spring is roughly V-shaped in the natural state, which includes a base and two branches extending from the base. Each branch is connected to a clamping arm 21. The elastic closing member 44 is configured so that the closing force of each clamping arm 21 is 8N to 12N, ensuring the stability of the two clamping arms 21 clamping the leaflets. Please refer to FIG. 10, in other embodiments, the elastic closing unit 42 is a torsion spring, which includes a spring body and two spring arms extending from the spring body, wherein one end of each spring arm away from the spring body is curled to form a connecting ring, which is sleeved on one end of the corresponding clamping arm 21, so that each spring arm is connected to a clamping arm 21 and provides a closing force of 3N to 10N. The elastic closing unit 42 can be made of elastic metal materials such as stainless steel, nickel titanium, etc.

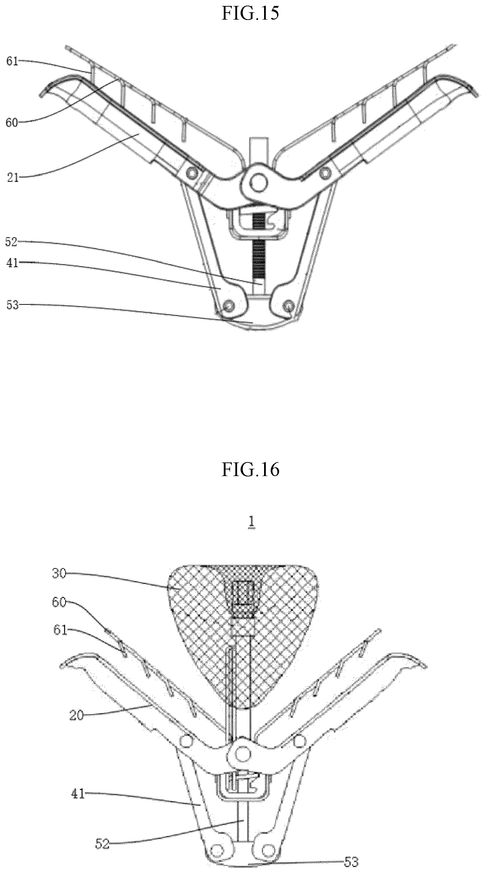

As shown in FIGs. 15 and 16, in one embodiment of the present disclosure, the driving part 40 includes a pair of connecting rods 41, a driving shaft 52 and a connecting seat 53, one end of each connecting rod 41 is rotatably connected to a clamping arm 21, the other end of each connecting rod 41 is rotatably connected to the connecting seat 53, one end of the driving shaft 52 is connected to the connecting seat 53, and the other end of the driving shaft 52 is movably installed in the supporting portion 10, and the driving shaft 52 can move along the axial direction of the supporting portion 10. When the driving shaft 52 slides axially in the supporting portion 10, the connecting rod 41 is configured to transmit the force of the driving shaft 52 to the clamping arm 21, so as to open and close the clamping arm 21.

Wherein, the material of the connecting rod 41 can be stainless steel, cobalt-chromium alloy, nickel-titanium alloy, etc., and in some embodiments, it is stainless steel, but the present disclosure does not limit it.

As shown in FIGs. 1 to 6 and 8, according to the valve clamping device 1 of the embodiment of the present disclosure, the surface (clamping surface) of the clamping arm 21 facing the adjusting portion 30 is provided with a plurality of protrusions 212. When the clamping arm 21 is closed relative to the adjusting portion 30, the end of the protrusion 212 can be embedded in the interior of the adjusting portion 30, thereby improving the clamping force of the clamping arm 21 and preventing the leaflet from slipping off.

As shown in FIGs. 1 to 8, according to the valve clamping device 1 of the embodiment of the present disclosure, the area (clamping surface) of the adjusting portion 30 opposite to the clamping arm 21 is provided with at least one group of first barbs 31, and the length of the first barbs 31 ranges from 0.3 mm to 3 mm. By providing the first barbs 31, the friction between the leaflet and the adjusting portion 30 can be increased, which can improve the clamping force during the clamping process on the one hand, and improve the stability after clamping on the other hand, and prevent the leaflet from slipping off. In some embodiments, the length of the first barb 31 ranges from 0.8 mm to 1.6 mm, but the present disclosure is not limited thereto.

As shown in FIGs. 1 to 8, in one embodiment of the present disclosure, the first barb 31 is inclined toward the direction close to the distal end of the supporting portion 10. When the valve clamping device 1 of this embodiment is used to capture the leaflet, the protrusion 212 provided on the clamping arm 21 captures one surface of the leaflet, and the first barb 31 provided on the outer surface of the adjusting portion 30 captures the other surface of the leaflet, the cooperation of the two can further enhance the capture ability of the valve clamping device 1, prevent the leaflet from slipping, and increase the friction between the clamping surface and the leaflet, thereby enhancing the stability of the leaflet clamping.

In one embodiment of the present disclosure, the angle between the first barb 31 and its tangent at the connection with the outer surface of the adjusting portion 30 is less than 90°. In this way, the capture force of the valve clamping device 1 on the leaflet can be improved or guaranteed. As shown in FIG. 7, the angle between the first barb 31 and the outer surface of the adjusting portion 30 is A, and A is less than 90°.

There are multiple first barbs 31, and the lengths of the multiple first barbs 31 can be set to be equal or can be increased in sequence in the direction gradually away from the distal end of the supporting portion 10. As shown in FIG.7, in one embodiment of the present disclosure, the length of the first barb 31 gradually increases in the direction gradually away from the distal end of the supporting portion 10. Since the anatomical structure of the leaflet presents a thin thickness at the leaflet edge, and then gradually thickens in the direction from the leaflet edge to the rough band of the leaflet, and the first barb 31 at the distal end of a group of first barbs 31 is embedded in the leaflet edge, and the first barb 31 at the proximal end is embedded in the rough band of the leaflet, in order to ensure that each first barb 31 can be smoothly embedded in the leaflet, the lengths of the multiple first barbs 31 are set to increase in sequence, so that the friction between the leaflet and the adjusting portion 30 can be better increased according to the anatomical structure of the leaflet to prevent the leaflet from slipping.

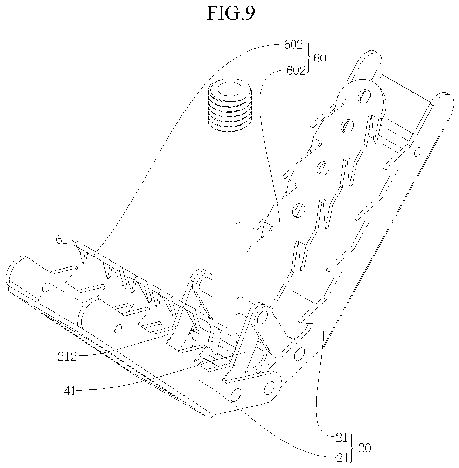

As shown in FIGs. 9, 10, 11, 15 and 16, according to the valve clamping device 1 of the embodiment of the present disclosure, the valve clamping device 1 also includes a gripper 60, and the gripper 60 includes a pair of elastic arms 602, each elastic arm 602 is respectively located between a clamping arm 21 and the adjusting portion 30, and the elastic arm 602 cooperates with the clamping arm 21 to clamp the leaflet. The gripper 60 cooperates with the clamping arm 21 to clamp the leaflet, thereby further improving the friction between the valve clamping device 1 and the leaflet, ensuring that the leaflet will not slip after being clamped by the valve clamping device 1, thereby improving the success rate of the surgery.

In some embodiments, the gripper 60 is at least partially made of shape memory material, and has been heat-set to have a specific shape, and the gripper 60 has a natural unfolded state and a folded state. When making the gripper 60, the shape memory material is first cut into a required shape by laser cutting, and then placed in a mold at about 550°C for heat setting to have a specific shape, but the present disclosure is not limited thereto. In a natural state, the elastic arm 602 extends radially outward in a direction away from the distal end of the supporting portion 10.

In some embodiments, the gripper 60 is made entirely of a superelastic nickel-titanium alloy, thereby providing elastic force for the gripper 60 to drive the elastic arm 602 toward the clamping arm 21 to clamp the tissue, and reduce the difficulty of the production process, simplify the process, and reduce production costs.

In one embodiment of the present disclosure, when the clamping arm 21 and the gripper 60 are both in an unfolded state, the angle between the two clamping arms 21 is smaller than the angle between the two elastic arms 602, that is, the elastic arm 602 is at least partially accommodated in the inner surface of the clamping arm 21, thereby ensuring that there is a certain clamping force between the elastic arm 602 and the clamping arm 21 to clamp the leaflet located therebetween.

In one embodiment of the present disclosure, the elastic arm 602 is provided with at least one set of second barbs 61 on the surface facing the clamping arm 21, so that the friction between the elastic arm 602 and the leaflet is increased by the second barbs 61, thereby further improving the friction between the valve clamping device 1 and the leaflet, ensuring that the leaflet will not slip after being clamped by the valve clamping device 1, thereby improving the success rate of the surgery.

### Performance test of valve clamping device

As shown in Table 1, nickel-titanium filament with a diameter of 0.20 mm is used to weave mesh structures 32 with different porosities by adjusting the weaving density, and then the adjusting portion 30 of each mesh structure 32 goes through heat-set under the same conditions and is respectively mounted on different supporting portions 10 and fixed, and then the other components of the valve clamping device 1 are assembled to obtain the valve clamping devices 1 of examples 1 to 5 and comparative examples 1 to 6, respectively. Except for the porosity of the adjusting portion 30, the other structures of all valve clamping devices 1 are exactly the same.

As shown in Table 2, nickel-titanium filaments with different diameters are used to weave the mesh structure 32 of the adjusting portion 30 with a porosity of 30% and 80%, respectively. Then, each adjusting portion 30 goes through heat-set under the same conditions and is respectively sleeved on different supporting parts 10 and fixed. Then, the other components of the valve clamping device are assembled to obtain the valve clamping devices of examples 6 to 7 and comparative examples 7 to 12, respectively. Except for the diameter of the adjusting portion 30, the other structures of all valve clamping devices 1 are exactly the same.

The deformation rate and radial support force of the valve clamping devices of examples 1 to 7 and comparative examples 1 to 12 are tested respectively, and it is verified whether the mitral regurgitation treatment effect and anti-fatigue performance are met respectively.

### Porosity test

The porosity of the mesh structure 32 of the adjusting portion 30 of the valve clamping device of each embodiment and comparative example is measured using a VHX-7000 microscope of Keyence Corporation, and the results are shown in Tables 1 and 2, respectively.

### Deformation rate test

First, the dimensions L1, W1, and T1 of the mesh structure 32 of the valve clamping device of each embodiment and the comparative example before force deformation are measured respectively using Kunshan Gaopin GP-300C high-definition CCD microscope. Then, the clamping portion 20 of each valve clamping device is used to clamp two transparent silicone pieces respectively to simulate the leaflets of the mitral valve, and then the clamping portion 20 is closed to make it close to the outside of the adjusting portion 30. Then, the dimensions L2, W2, and T2 of the adjusting portion 30 of each valve clamping device in three directions are measured respectively. Finally, the calculation formula: ΔL=(L2-L1)/L1; ΔW= (W2-W1)/W1; ΔT = (T1-T2)/T1; ΔS = ΔL*ΔW*ΔT is used to calculate the deformation rate of the mesh structure 32 of the adjusting portion 30 of each valve clamping device, and the results are shown in Table 1 and Table 2.

### Radial support force test

According to laboratory tests, the reasonable range of the radial support force of the adjusting portion 30 is 0.8-3.2N, when the radial support force is lower than 0.8N, the leaflet is easy to slip off, and when the radial support force is greater than 3.2N, the radial support force will act in the opposite direction on the elastic arm 602 (i.e. offset part of the elastic closing force), causing the valve clamping device to be intolerant to fatigue.

The mesh structure 32 of the valve clamping device of each embodiment and comparative example is tested respectively for the reverse force (radial support force) that can be provided correspondingly when subjected to the same external force (clamping force) and deformed, so as to determine whether the valve clamping device of each embodiment and comparative example can meet the radial support force requirements.

Testing method: First fix the supporting portion 10 of the valve clamping device, and let the clamping portion 20 be in the unfolded state, and use two Tension Meters to simulate the clamping arm to compress the adjusting portion 30 in the clamping direction of the clamping arm. When the thickness of the adjusting portion 30 reaches T2, record the maximum value of the Tension Meters. The testing equipment is the Japanese IMADA DST-200N. The results are shown in Tables 1 and 2.

### Test of the effect of mitral regurgitation treatment

The method of the effect of mitral regurgitation treatment refers to the relevant content of "YY/T 1449.3-2016 Cardiovascular Implant Artificial Heart Valve Part 3 Transcatheter Implantable Artificial Heart Valve". When the total mitral regurgitation percentage (accounting for forward flow) is ≤20%, the valve clamping device is considered to meet the use requirements.

The test equipment is the MPD-1000 modular artificial heart valve pulsating flow tester of Shanghai XinBan Testing Equipment Co., Ltd. The test parameters are as follows: the number of samples: n=12; the number of cycles per minute is 70 times; the simulated cardiac output is 5L/min; the aortic pressure is 100mmHg; the sample is a simulated mitral valve, the leaflet thickness is 1mm, the hardness is 30A, the valve ring edge is blocked to prevent paravalvular leakage, and the distance between the anterior and posterior leaflets is 13mm. Sample processing: the valve clamping device of each embodiment and comparative example is used to clamp the anterior and posterior leaflets of the mitral valve model respectively, and is placed on the pulsating flow tester to simulate the mitral valve being clamped. Then, the percentage of the regurgitation volume reduction is calculated by monitoring the flow meter. When the total regurgitation percentage (accounting for the forward flow) is ≤20%, the valve clamping device is considered to meet the use requirements. The results are shown in Tables 1 and 2.

### Fatigue test

The purpose of the fatigue test is to test whether the valve clamping device of each embodiment and comparative example can meet the load of 10 years of implantation as a medical device implant. The test equipment is the Enduratec ELF 3200 high-load dynamic mechanical analyzer of TA. Instruments, USA. The test parameters are as follows: number of samples: n=12, cyclic tension (peak): 4.00N±0.30N, amplitude: 1mm, frequency: 50Hz, water bath temperature: 37 °C ±0.5°C, cycle: ≥400 million times. A non-destructive fatigue test was performed on the valve clamping device 1 of each embodiment and comparative example, and the slippage and wear of each valve clamping device caused by load during the fatigue test cycle were recorded. The results are shown in Tables 1 and 2.

**Table 1 Performance test results of valve clamping devices of embodiments 1 to 5 and comparative examples 1 to 6**

| No. | poro -sity | diam -eter | before clamping (unit: mm) | | | after clamping (unit: mm) | | | deformation rate | | | | fill the gap | radial support force < unit: N) | anti-fatigue |
|---|---|---|---|---|---|---|---|---|---|---|---|---|---|---|---|
| | | | length L1 | width W1 | thickness T1 | length L2 | width W2 | thickness T2 | ΔL | ΔW | ΔT | ΔS | | | |
| comparative example 1 | 20% | 0.20 | 10.04 | 6.03 | 6.06 | 10.72 | 6.15 | 5.12 | 0.0677 | 0.0199 | 0.1551 | 0.0002 | yes | 3.81 | no |
| comparative example 2 | 23% | 0.20 | 10.05 | 6.05 | 6.04 | 10.86 | 6.19 | 4.85 | 0.0806 | 0.0231 | 0.1970 | 0.0004 | yes | 3.68 | no |
| comparative example 3 | 26% | 0.20 | 10.04 | 6.04 | 6.06 | 11.03 | 6.22 | 4.63 | 0.0986 | 0.0298 | 0.2360 | 0.0006 | yes | 3.42 | no |
| embodiment 1 | 30% | 0.20 | 10.06 | 6.05 | 6.03 | 11.06 | 6.23 | 4.53 | 0.0994 | 0.0299 | 0.2488 | 0.0007 | yes | 3.29 | yes |
| embodiment 2 | 40% | 0.20 | 10.04 | 6.04 | 6.05 | 11.23 | 6.31 | 4.20 | 0.1185 | 0.0447 | 0.3058 | 0.0016 | yes | 2.76 | yes |
| embodiment 3 | 50% | 0.20 | 10.08 | 6.03 | 6.04 | 11.46 | 6.42 | 3.88 | 0.1369 | 0.0647 | 0.3576 | 0.0032 | yes | 2.44 | yes |
| embodiment 4 | 60% | 0.20 | 10.06 | 6.06 | 6.03 | 11.66 | 6.62 | 3.42 | 0.1590 | 0.0924 | 0.4328 | 0.0064 | yes | 2.21 | yes |
| embodiment 5 | 80% | 0.20 | 10.02 | 6.05 | 6.03 | 12.03 | 6.80 | 2.11 | 0.2006 | 0.1240 | 0.6501 | 0.0162 | yes | 2.02 | yes |
| comparative example 4 | 82% | 0.20 | 10.07 | 6.02 | 6.08 | 12.23 | 6.92 | 2.11 | 0.2145 | 0.1495 | 0.6530 | 0.0209 | no | 1.96 | yes |
| comparative example 5 | 84% | 0.20 | 10.08 | 6.03 | 6.06 | 12.39 | 6.98 | 2.04 | 0.2292 | 0.1575 | 0.6634 | 0.0240 | no | 1.87 | yes |
| comparative example 6 | 86% | 0.20 | 10.03 | 6.04 | 6.03 | 12.50 | 7.06 | 1.92 | 0.2463 | 0.1689 | 0.6816 | 0.0283 | no | 1.79 | yes |

It can be seen from Table 1 that:
1) In comparative examples 1 to 3, the porosity of the mesh structure 32 of the adjusting portion 30 is less than 30%. At this time, the weaving density of the mesh structure 32 is relatively high, which can meet the therapeutic effect of mitral regurgitation. However, due to the small deformation rate of the mesh structure 32, the radial support force is too small, and the valve clamping device 1 cannot effectively clamp the leaflet for a long time. The leaflet is easy to slip off, and the anti-fatigue performance of the valve clamping device 1 is poor;
2) In comparative examples 4 to 6, the porosity of the adjusting portion 30 is greater than 80%. The weaving density of adjusting portion 30 is relatively large, and the therapeutic effect on mitral regurgitation does not meet the requirements;
3) In embodiments 1 to 5, the porosity range of the mesh structure 32 of the adjusting portion 30 is 30% to 80%. At this time, the adjusting portion 30 can effectively reduce the central mitral regurgitation of the valve clamping device 1, and the therapeutic effect of the valve clamping device on mitral regurgitation meets the requirements; at the same time, the deformation rate ΔS of the mesh structure 32 of the adjusting portion 30 is reasonable, and the adjusting portion 30 can provide appropriate radial support force to the leaflets and the clamping arms 21, which can not only effectively clamp the leaflets, but also meet the anti-fatigue performance of the implant.

**Table 2 Performance test results of the valve clamping device of examples 6 to 7 and comparative examples 7 to 12**

| No. | poro -sity | diam -eter | before clamping (unit: mm) | | | after clamping (unit: mm) | | | deformation rate | | | | fill the gap | radial support force (unit: N) | anti-fatigue |
|---|---|---|---|---|---|---|---|---|---|---|---|---|---|---|---|
| | | | length L1 | width W1 | thick -ness T1 | length L2 | width W2 | thickness T2 | ΔL | ΔW | ΔT | ΔS | | | |
| comparative example 7 | 30% | 0.03 | 10.01 | 6.05 | 6.00 | 11.84 | 6.42 | 3.12 | 0.1828 | 0.0612 | 0.4800 | 0.0054 | yes | 0.76 | no |
| embodiment 6 | 30% | 0.06 | 10.03 | 6.02 | 6.03 | 11.75 | 6.36 | 3.22 | 0.1715 | 0.0565 | 0.4660 | 0.0045 | yes | 0.81 | no |
| comparative example 8 | 30% | 0.30 | 10.12 | 6.09 | 6.05 | 10.66 | 6.24 | 4.30 | 0.0534 | 0.0246 | 0.2893 | 0.0004 | yes | 412 | no |
| comparative example 9 | 30% | 0.40 | 10.13 | 6.09 | 6.07 | 10.52 | 6.24 | 4.71 | 0.0385 | 0.0246 | 0.2241 | 0.0002 | yes | 4.06 | no |
| comparative example 10 | 80% | 0.04 | 10.00 | 6.03 | 6.03 | 12.41 | 7.02 | 3.54 | 0.2410 | 0.1642 | 0.4129 | 0.0163 | yes | 0.08 | no |
| comparative example 11 | 80% | 0.05 | 10.05 | 6.04 | 6.04 | 12.38 | 6.94 | 3.62 | 0.2318 | 0.1490 | 0.4007 | 0.0138 | yes | 0.10 | no |
| embodiment 7 | 80% | 0.20 | 10.11 | 6.05 | 6.03 | 11.82 | 6.34 | 4.36 | 0.1691 | 0.0479 | 0.2769 | 0.0022 | yes | 320 | yes |
| comparative example 12 | 80% | 0.30 | 10.13 | 6.08 | 6.09 | 11.74 | 6.22 | 4.41 | 0.1589 | 0.0230 | 0.2759 | 0.0010 | yes | 3.28 | no |

It can be seen from Table 2 that:
1) With the porosity is the same, both are 30%, when the diameter of the nickel-titanium filament in example 6 is 0.06mm, the deformation rate of the mesh structure, the mitral regurgitation treatment effect, the radial support force and the anti-fatigue performance all meet the requirements; in comparative example 7, since the diameter of the nickel-titanium filament is too small, the nickel-titanium filament is easy to break and does not meet the anti-fatigue performance; in comparative examples 8 and 9, since the diameter of the nickel-titanium filament is too large, the radial support force is too large. Although sufficient clamping force can be provided, the clamping arm 21 is subjected to a large reverse force for a long time, which affects the anti-fatigue performance;
2) With the porosity is the same, both are 80%, when the diameter of the nickel-titanium filament in example 7 is 0.20 mm, the deformation rate of the mesh structure, the mitral regurgitation treatment effect, the radial support force and the anti-fatigue performance can all meet the requirements; while in comparative examples 10 and 11, although the mitral regurgitation treatment effect can meet the requirements, the nickel-titanium filament is easy to break due to the small diameter of the nickel-titanium filament, which does not meet the anti-fatigue performance; and in comparative example 12, due to the excessive diameter of the nickel-titanium filament, its radial support force is too large, so the clamping arm 21 is subjected to a large reverse force for a long time and is not anti-fatigue.

From the performance test results of the valve clamping device in Tables 1 and 2, it can be seen that in the present disclosure, the deformation degree of the adjusting portion 30 in the thicknesswise direction is greater than the deformation degree of the adjusting portion 30 in the lengthwise direction. The deformation degree of the adjusting portion 30 in the widthwise direction enables the adjusting portion to provide a suitable radial support force to clamp the valve leaflet, and improve the anti-fatigue performance of the device, while effectively blocking the central mitral regurgitation of the clamping gap. The deformation rate of the adjusting portion 30 in the thicknesswise direction ranges from 25% to 65%, which can provide appropriate support and clamping force, and avoid leaflet damage caused by excessive pulling of the leaflets; the deformation rate of the adjusting portion 30 in the lengthwise direction ranges from 10% to 20%, so that the adjusting portion 30 can provide appropriate support and prevent exposure; the deformation rate of the adjusting portion 30 in the widthwise direction ranges from 3% to 12%, which can provide appropriate support force and will not affect the overall outer diameter of the valve clamping device 1 after closing, so as to facilitate delivery through a catheter. The porosity of the mesh structure 32 of the adjusting portion 30 ranges from 30% to 80%, so that the adjusting portion 30 can effectively fill the gap between the clamping arms 21 of the valve clamping device 1 (i.e., the leaflet gap when the leaflets are clamped each time they are closed), and the adjusting portion 30 is deformed to a certain extent after being compressed to provide appropriate radial support force for the clamping arms 21, and does not affect the anti-fatigue performance of the valve clamping device 1. The diameter of the filament 320 ranges from 0.06mm to 0.20mm, so that the filament 320 is easy to be woven, has sufficient strength to prevent breakage, and can provide appropriate radial support force.

In the description of the present disclosure, it should be understood that the terms "upper", "lower", "left", "right", "inner", "outer", "axial", "radial", "circumferential" and the like indicate the orientation or position relationship based on the orientation or position relationship shown in the accompanying drawings, which is only for the convenience of describing the present disclosure and simplifying the description, and does not indicate or imply that the device or element referred to must have a specific orientation, be constructed and operated in a specific orientation, and therefore cannot be understood as a limitation on the present disclosure. In addition, the features defined as "first" and "second" may explicitly or implicitly include one or more of the features. In the description of the present disclosure, unless otherwise specified, "multiple" means two or more.

In the description of the present disclosure, it should be noted that, unless otherwise clearly specified and limited, the terms "installed", "connected", and "connected" should be understood in a broad sense, for example, it can be a fixed connection, a detachable connection, or an integral connection; it can be a mechanical connection or an electrical connection; it can be directly connected, or indirectly connected through an intermediate medium, or it can be the internal connection of two elements. For ordinary technicians in this field, the specific meanings of the above terms in the present disclosure can be understood in specific circumstances.

In the description of this specification, the description of the reference terms "one embodiment", "some embodiments", "illustrative embodiments", "examples", "specific examples", or "some examples" means that the specific features, structures, materials, or characteristics described in conjunction with the embodiment or example are included in at least one embodiment or example of the present disclosure. In this specification, the schematic representation of the above terms does not necessarily refer to the same embodiment or example. Moreover, the specific features, structures, materials, or characteristics described can be combined in any one or more embodiments or examples in a suitable manner.

Although the embodiments of the present disclosure have been shown and described, it will be appreciated by those skilled in the art that various changes, modifications, substitutions and variations may be made to the embodiments without departing from the principles and spirit of the present disclosure, and the scope of the present disclosure is defined by the claims and their equivalents.

## Claims

1. A valve clamping device, comprising:
a supporting portion having a certain length;
a clamping portion comprising a pair of clamping arms that can be opened and closed relative to each other; and
an adjusting portion; wherein the adjusting portion is arranged outside the supporting portion, the adjusting portion is deformable, the adjusting portion is a three-dimensional structure with a certain length, width and thickness, and the pair of the clamping arms are closed relative to the adjusting portion so that the adjusting portion is deformed, and the deformation degree of the adjusting portion in the thicknesswise direction>the deformation degree of the adjusting portion in the lengthwise direction>the deformation degree of the adjusting portion in the widthwise direction.

2. The valve clamping device according to claim 1, wherein a deformation rate of the adjusting portion in the thicknesswise direction ranges from 25% to 65%.

3. The valve clamping device according to claim 1, wherein a deformation rate of the adjusting portion in the lengthwise direction ranges from 10% to 20%.

4. The valve clamping device according to claim 1, wherein a deformation rate of the adjusting portion in the widthwise direction ranges from 3% to 12%.

5. The valve clamping device according to claim 1, wherein the adjusting portion is made of shape memory material and has been heat-set.

6. The valve clamping device according to claim 1, wherein the adjusting portion includes a mesh structure, and a porosity of the mesh structure ranges from 30% to 80%.

7. The valve clamping device according to claim 6, wherein the mesh structure is woven by a plurality of filaments, and a diameter of each of the filaments is in a range from 0.06 mm to 0.20 mm.

8. The valve clamping device according to claim 7, wherein the weaving manner of the filament is selected from at least one of a single-strand weaving manner, a double-strand weaving manner or a mixed weaving manner of single and double strands.

9. The valve clamping device according to claim 1, wherein one end of the adjusting portion is fixedly connected with the supporting portion, and the other end is free relative to the supporting portion.

10. The valve clamping device according to claim 1, wherein the valve clamping device further includes a driving part configured to drive the relative opening and closing of the pair of clamping arms.

11. The valve clamping device according to claim 10, wherein the driving part includes a pair of connecting rods and an elastic closing unit, one end of each of the connecting rods is rotatably connected to one of the clamping arms, and the other end of the each connecting rods is connected to the supporting portion, one end of the elastic closing unit is connected to the connecting rod, and the other end of the elastic closing unit is connected to the clamping arm.

12. The valve clamping device according to claim 10, wherein the driving part includes a pair of connecting rods, a driving shaft and a connecting seat, one end of each of the connecting rods is rotatably connected to one of the clamping arms, and the other end of each of the connecting rods is rotatably connected to the connecting seat, one end of the driving shaft is connected to the connecting seat, and the other end of the driving shaft is movably fitted in the supporting portion.

13. The valve clamping device according to claim 1, wherein a surface of the clamping arm facing the adjusting portion is provided with a plurality of protrusions, and when the clamping arm is closed relative to the adjusting portion, the protrusions abut against the adjusting portion.

14. The valve clamping device according to claim 1, wherein the adjusting portion has at least one set of first barbs provided on an area thereof opposite to the clamping arm, and a length of each of the first barbs is in a range from 0.3mm to 3mm.

15. The valve clamping device according to claim 14, wherein the first barb is inclined in a direction toward a distal end of the supporting portion.

16. The valve clamping device according to claim 14 or 15, wherein the included angle between the first barb and the tangent at the connection with the outer surface of the adjusting portion is less than 90°.

17. The valve clamping device according to one of claims 14 to 16, wherein along the direction of getting away from the distal end of the supporting portion, the length of the first barb gradually increases.

18. The valve clamping device according to one of claims 1 to 17, wherein the valve clamping device further includes a gripper, the gripper includes a pair of elastic arms, each of the elastic arms is located between one of the clamping arms and the adjusting portion, and the elastic arm and the clamping arm cooperate to clamp a leaflet.

19. The valve clamping device according to claim 18, wherein when both the clamping arms and the gripper are in the unfolded state, the angle between the two clamping arms is smaller than the angle between the two elastic arms.

20. The valve clamping device according to claim 18 or 19, wherein the elastic arm is provided with at least one set of second barbs on the surface facing the clamping arm.
